# EUROPÄISCHE PATENTANMELDUNG

(11) **EP 3 679 907 A1**
(43) Veröffentlichungstag der Anmeldung: **15.07.2020**
(21) Anmeldenummer: 19151373.8
(22) Anmeldetag: 11.01.2019
(51) Int. Cl.: A61F 5/44, A61M 25/00, A61F 5/453

(54) **VORLAGE FÜR DIE ANWENDUNG BEI EINEM HARNKATHETER FÜR DEN MANN**

(71) Anmelder: Rudolf Heintel Gesellschaft m.b.H., 1030 Wien (AT)
(72) Erfinder: BAUER, Wilhelm, 3400 Klosterneuburg (AT); PÖLTENSTEIN, Markus, 1030 Wien (AT)
(74) Vertreter: Sonn & Partner Patentanwälte

(57) **Zusammenfassung**

Die Erfindung betrifft eine Vorlage (1) für die Anwendung bei einem Harnkatheter (2) für den Mann, mit einem Element (3) aus saugfähigem Material (4) und einer flüssigkeitsdichten Schicht (5) an einer Seite, und zumindest einem Klebeelement (6) zum Befestigen des Elements (3) in einer Gebrauchslage und zur Befestigung des Elements (3) in seiner Gebrauchslage am Harnkatheter (2). Erfindungsgemäß ist das Element (3) aus saugfähigem Material (4) kreissektorförmig ausgebildet und für seine Gebrauchslage zu einem Trichter zusammenrollbar und mit dem zumindest einen Klebeelement (6) fixierbar, und an einem Kreisradius (r) des Kreissektors zumindest ein weiteres Klebeelement (7) zur Befestigung am Harnkatheter (2) angeordnet, welches weitere Klebeelement (7) ein klebstofffreies Ende (8) aufweist.

## Beschreibung

Die Erfindung betrifft eine Vorlage für die Anwendung bei einem Harnkatheter für den Mann, mit einem Element aus saugfähigem Material und einer flüssigkeitsdichten Schicht an einer Seite, und zumindest einem Klebeelement zum Befestigen des Elements in einer Gebrauchslage und zur Befestigung des Elements in seiner Gebrauchslage am Harnkatheter.

Harnkatheter oder transurethrale Blasenkatheter werden über die Harnröhre in die Blase eingebracht und dort über einen aufblasbaren Ballon gehalten. Je nach Einsatzzweck unterscheidet man zwischen transurethralen Dauer- und Einmalkathetern. Die Länge der Katheter wird üblicherweise in Zentimetern angegeben, die Dicke in Charriere (1 Ch = 1/3 mm).

Insbesondere bei der Anwendung von Harnkathetern bei männlichen Patienten kommt es zu einem Austreten von Harn an der Außenseite des Katheters und in der Folge zu Verschmutzungen. Darüber hinaus stellen Infektionen eine weitere Komplikation dar.

Zur Vermeidung oder Minimierung dieses Problems werden Sammelbeutel oder dergl. am äußeren Ende des Harnkatheters angeschlossen.

Beispielsweise beschreibt die DE 10 2012 020 721 A1 einen Katheter, bei dem zur Vermeidung von Infektionen am äußeren Teil des Katheterschlauchs eine flächige Vorlage aus einem flüssigkeitsbindenden Material angeordnet ist. Die Vorlage nimmt Harn, der an der Außenseite des Katheters austritt, auf. Die Fixierung der Vorlage kann mit einer auf den Schlauch aufsteckbaren Klemme oder mit einem Klebeband erfolgen.

Die US 5,980,507 A beschreibt einen Aufsatz für einen Harnkatheter für den Mann zur Aufnahme von Urin, mit einem saugfähigen Körper und einer Befestigungseinheit, welche den saugfähigen Körper mittels Klebestreifen lösbar um den Katheterschlauch anordnet. Zusätzlich ist eine Fixierung der Vorlage am Katheterschlauch mit Klebestreifen möglich.

Bisherige Vorlagen für die Anwendung bei einem Harnkatheter für den Mann sind meist aufwändig in der Herstellung und bzw. oder in der Anwendung. Es besteht daher ein Bedarf an einem vergleichsweise kostengünstig und einfach herstellbaren Produkt.

Die Aufgabe der vorliegenden Erfindung besteht daher in der Schaffung einer oben genannten Vorlage für die Anwendung bei einem Harnkatheter für den Mann, welche möglichst einfach und kostengünstig herstellbar ist. Die Vorlage soll möglichst rasch und einfach anwendbar und auch wieder entfernbar sein, um eine breite Verwendung garantieren und die Hygienebedingungen einhalten zu können. Nachteile bekannter Produkte sollen vermieden oder zumindest reduziert werden.

Gelöst wird die erfindungsgemäße Aufgabe dadurch, dass das Element aus saugfähigem Material kreissektorförmig ausgebildet und für seine Gebrauchslage zu einem Trichter zusammenrollbar und mit dem zumindest einen Klebeelement fixierbar ist, und dass an einem Kreisradius des Kreissektors zumindest ein weiteres Klebeelement zur Befestigung am Harnkatheter angeordnet ist, welches weitere Klebeelement ein klebstofffreies Ende aufweist. Durch diese Gestaltung der Vorlage ist eine einfache und kostengünstige Herstellung garantiert, da der Hauptbestandteil der Vorlage, das kreissektorförmige Element aus saugfähigem Material einfach aus einer ebenen Materialbahn hergestellt werden kann. Das Applizieren der Klebeelemente am saugfähigen Material kann ebenfalls besonders rasch und einfach auch automatisiert vorgenommen werden. Die Anwendung der Vorlage ist ebenfalls durch Formen des kreissektorförmigen Elements um den Katheter zu einem Trichter und Fixieren des Trichters durch das Klebeband möglich. Mit dem zumindest einen weiteren Klebeelement kann der Trichter an der gewünschten Stelle am Katheterschlauch befestigt werden. Durch das klebstofffreie Ende des zumindest einen weiteren Klebeelements kann dieses wieder sehr rasch und einfach entfernt und die Vorlage entsorgt bzw. ausgetauscht werden. Die erfindungsgemäße Vorlage zeichnet sich durch besonders kostengünstige Herstellbarkeit und einfache Anwendbarkeit aus. Natürlich sind Abweichungen des Elements aus saugfähigem Material von der reinen Kreissektorform möglich. So kann das saugfähige Material auch in Gestalt eines Teils eines Polygons gestaltet sein.

Vorteilhafterweise weist das kreissektorförmige Element einen Mittelpunktswinkel des Kreissektors von 90° bis 270°, vorzugsweise 180° auf. Je nach Größe bzw. Öffnungswinkel des Trichters, der aus dem kreissektorförmigen Element gebildet wird, kann der Mittelpunktswinkel entsprechend gewählt werden. Beim bevorzugten Mittelpunktswinkel von 180° kann der Verschnitt bei der Herstellung des Elements aus dem saugfähigen Material besonders gering gehalten werden.

Idealerweise beträgt der Kreisradius des kreissektorförmigen Elements zwischen 5 cm und 10 cm. Diese Abmessungen haben sich bei der Anwendung der Vorlage bei einem Harnkatheter für den Mann als besonders geeignet herausgestellt.

Der Flächeninhalt des kreissektorförmigen Elements beträgt vorzugsweise zwischen 20 cm² und 200 cm². Zusätzlich zur Dicke des saugfähigen Materials bestimmt die Fläche des kreissektorförmigen Elements die Aufnahmefähigkeit für den aufzunehmenden Harn.

Wenn im Bereich des Mittelpunkts des kreissektorförmigen Elements eine Aussparung vorgesehen ist, kann der Harnkatheter in der Gebrauchslage der Vorlage ideal umfasst werden und es können Falten vermieden werden, über welche an der Außenseite des Katheterschlauchs fließender Harn austreten kann.

Die Aussparung ist vorzugsweise kreisförmig ausgebildet mit einem Radius von vorzugsweise 3 mm bis 11 mm entsprechend den üblichen Durchmessern von Harnkathetern für den Mann im Bereich von 11 bis 34 Charrière.

Anstelle einer Aussparung kann im Bereich des Mittelpunkts des kreissektorförmigen Elements auch zumindest ein Einschnitt vorgesehen sein. Durch zumindest einen solchen Einschnitt kann ebenfalls eine verbesserte Anbindung der Vorlage am Katheter erzielt werden. Ein Einschnitt ist besonders einfach herstellbar.

Dieser zumindest eine Einschnitt weist entsprechend der oben genannten üblichen Katheterdurchmesser vorzugsweise eine Tiefe von 3 mm bis 11 mm auf.

Das kreissektorförmige Element kann aus hochabsorbierenden Kunststofffasern, insbesondere Acryl- oder Polyesterfasern, gebildet sein. Derartige Materialien sind von Wundverbänden bekannt und ermöglichen die Aufnahme relativ großer Mengen an Körperflüssigkeiten.

Zusätzlich kann im kreissektorförmigen Element eine Gel-bildenden Substanz angeordnet sein, welche den aufgenommenen Harn bindet.

An der der flüssigkeitsdichten Schicht gegenüberliegenden Seite des kreissektorförmigen Elements kann ein Kontaktnetz angeordnet sein. Ein solches Kontaktnetz weist Poren bzw. Öffnungen zur Aufnahme des Harns auf und verhindert aber ein Festkleben der Haut am absorbierenden Material.

Das Klebeelement zum Befestigen des kreissektorförmigen Elements in seiner Gebrauchslage kann beispielsweise durch eine Rundung gebildet sein. Die Form ist so gestaltet, dass beim Einrollen des kreissektorförmigen Elements zu einem Trichter keine Überlappung oder ein Überstand entsteht. Das Klebeelement muss so groß gestaltet werden, damit die Fixierung des Trichters am Katheter möglichst rasch und einfach möglich wird.

Die Klebeelemente, also das Klebeelement zum Befestigen des kreissektorförmigen Elements in der Gebrauchslage und das weitere Klebeelement zur Befestigung am Harnkatheter können einstückig ausgebildet sein. Dies erleichtert die Herstellung der Vorlage und reduziert dadurch auch die Herstellungskosten.

Die Dicke des kreissektorförmigen Elements kann 1 bis 9 mm, vorzugsweise 3 bis 3,5 mm betragen. Die Dicke hängt davon ab, wie viele Lagen an saugfähigem Material verarbeitet werden.

Die vorliegende Erfindung wird anhand der beigefügten Zeichnungen näher erläutert. Darin zeigen:
- Fig. 1: eine Draufsicht auf eine Ausführungsform einer Vorlage für die Anwendung bei einem Harnkatheter für den Mann;
- Fig. 2: ein Schnittbild durch das kreissektorförmige Element der Vorlage entlang der Schnittlinie II - II in Fig. 1;
- Fig. 3A: ein Detail der Vorlage im Bereich des Mittelpunkts M des kreissektorförmigen Elements mit einer Aussparung;
- Fig. 3B: ein Detail der Vorlage im Bereich des Mittelpunkts M des kreissektorförmigen Elements mit einem Einschnitt; und
- Fig. 4: die Vorlage gemäß Fig. 1 in ihrer Gebrauchslage am Harnkatheter für den Mann befestigt.

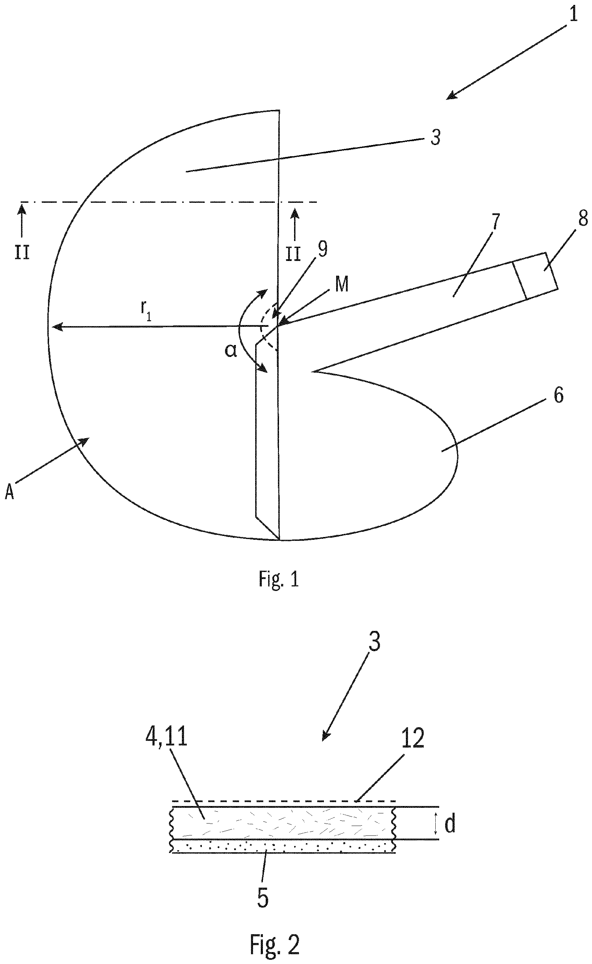

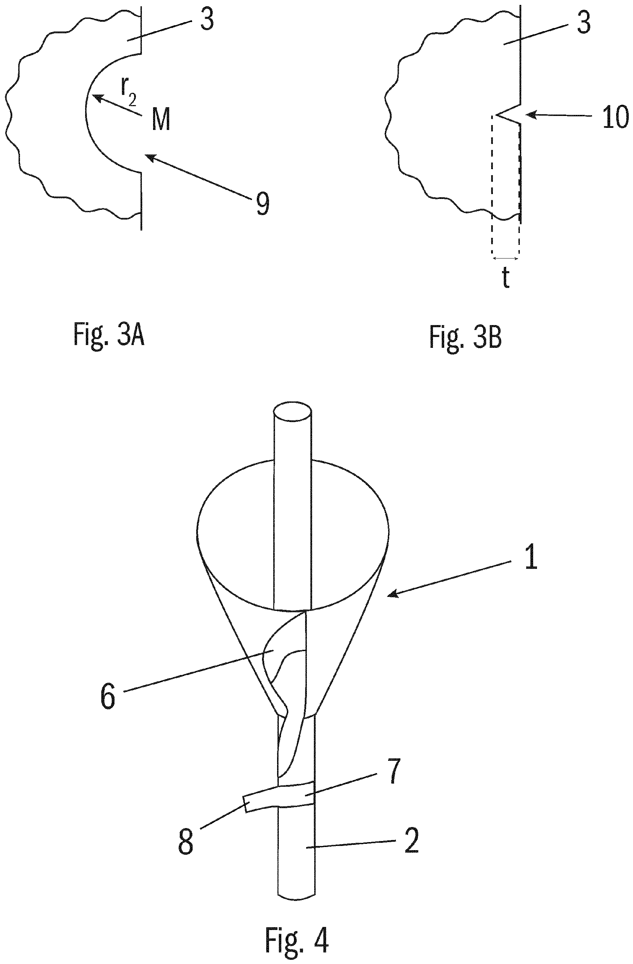

Fig. 1 zeigt eine Draufsicht auf eine Ausführungsform einer Vorlage 1 für die Anwendung bei einem Harnkatheter 2 für den Mann. Die Vorlage 1 weist ein kreissektorförmiges Element 3 auf, welches im dargestellten Ausführungsbeispiel einen Mittelpunktswinkel α von 180° aufweist. Andere Mittelpunktswinkel α zwischen 90° und 270° sind ebenfalls möglich. Geeignete Größen für das kreissektorförmige Element 3 umfassen einen Kreisradius r₁ zwischen 5 cm und 10 cm und einen Flächeninhalt A zwischen 20 cm² und 200 cm². Über zumindest ein Klebeelement 6 kann die Vorlage 1 bzw. das kreissektorförmige Element 3 zu einem Trichter geformt und in der Gebrauchslage befestigt werden (siehe Fig. 4). Mit zumindest einem weiteren Klebeelement 7, welches mit dem Klebeelement 6 einstückig ausgebildet sein kann, erfolgt eine Befestigung der Vorlage am Harnkatheter 2. Zur leichteren Enfernung der Vorlage 1 vom Harnkatheter weist das weitere Klebeelement 7 ein klebstofffreies Ende 8 auf. Die Klebeschichten der Klebelemente 6, 7 werden in üblicher Weise mit einer Abziehfolie (nicht dargestellt) geschützt, welche vor der Anwendung abgezogen wird.

In Fig. 2 ist ein Schnittbild durch das kreissektorförmige Element 3 der Vorlage 1 entlang der Schnittlinie II - II in Fig. 1 dargestellt. Dementsprechend besteht das kreissektorförmige Element 3 der Vorlage 1 aus saugfähigem Material 4 und einer flüssigkeitsdichten Schicht 5 an einer Seite. An der der flüssigkeitsdichten Schicht 5 gegenüberliegenden Seite kann ein Kontaktnetz 12 angeordnet sein, welches die Aufnahme von Flüssigkeit in die Schicht aus saugfähigem Material 4 zulässt, aber ein Anhaften oder Kleben am saugfähigen Material 4 verhindert. Das saugfähige Material 4 des kreissektorförmigen Elements 3 kann aus hochabsorbierenden Kunststofffasern, insbesondere Acryl- oder Polyesterfasern, gebildet sein. Darüber hinaus kann eine Gel-bildende Substanz 11 im saugfähigen Material angeordnet sein. Die Dicke d des kreissektorförmigen Elements 3 beträgt beispielsweise 1 mm bis 9 mm, vorzugsweise 3 mm bis 3,5 mm.

Fig. 3A zeigt ein Detail der Vorlage 1 im Bereich des Mittelpunkts M des kreissektorförmigen Elements 3 mit einer kreisförmigen Aussparung 9. Die Aussparung 9 ist vorzugsweise kreisförmig ausgebildet ist mit einem Radius r₂ von vorzugsweise 1 mm bis 11 mm entsprechend gängiger Durchmesser des Harnkatheters 2 von 11 bis 34 Charriere.

Fig. 3B zeigt eine Variante eines Einschnitts 10 im Bereich des Mittelpunkts M des kreissektorförmigen Elements 3. Dieser Einschnitt 10 weist vorzugsweise eine Tiefe t von 1 mm bis 11 mm auf.

Schließlich zeigt Fig. 4 die Vorlage 1 gemäß Fig. 1 in ihrer Gebrauchslage am Harnkatheter 2 für den Mann befestigt. Bei der Anwendung der Vorlage 1 wird das kreissektorförmige Element 3 um den Harnkatheter 2 angeordnet und mit dem Klebeelement 6 die Trichterform fixiert. Nachdem die Vorlage an die gewünschte Stelle am Harnkatheter 2 verschoben wurde, erfolgt die Fixierung am Harnkatheter in dem zumindest einen weiteren Klebeelement 7. Dadurch, dass das zumindest eine weitere Klebeelement 7 ein klebstofffreise Ende 8 aufweist, lässt sich die Vorlage auch wieder besonders rasch und einfach vom Harnkatheter 2 lösen und entfernen.

Die beschriebene Vorlage 1 für die Anwendung beim Harnkatheter 2 für den Mann lässt sich besonders einfach und kostengünstig herstellen und besonders einfach anwenden. Aus diesem Grund trägt die Vorlage 1 zur Verbesserung der Hygiene und Vermeidung von Infektionen bei.

## Patentansprüche

1. Vorlage (1) für die Anwendung bei einem Harnkatheter (2) für den Mann, mit einem Element (3) aus saugfähigem Material (4) und einer flüssigkeitsdichten Schicht (5) an einer Seite, und zumindest einem Klebeelement (6) zum Befestigen des Elements (3) in einer Gebrauchslage und zur Befestigung des Elements (3) in seiner Gebrauchslage am Harnkatheter (2), **dadurch gekennzeichnet, dass** das Element (3) aus saugfähigem Material (4) kreissektorförmig ausgebildet und für seine Gebrauchslage zu einem Trichter zusammenrollbar und mit dem zumindest einen Klebeelement (6) fixierbar ist, und dass an einem Kreisradius (r) des Kreissektors zumindest ein weiteres Klebeelement (7) zur Befestigung am Harnkatheter (2) angeordnet ist, welches weitere Klebeelement (7) ein klebstofffreies Ende (8) aufweist.

2. Vorlage (1) nach Anspruch 1, **dadurch gekennzeichnet, dass** das kreissektorförmige Element (3) einen Mittelpunktswinkel (α) des Kreissektors von 90° bis 270°, vorzugsweise 180° aufweist.

3. Vorlage (1) nach Anspruch 1 oder 2, **dadurch gekennzeichnet, dass** der Kreisradius (r₁) des kreissektorförmigen Elements (3) zwischen 5 cm und 10 cm beträgt.

4. Vorlage (1) nach einem der Ansprüche 1 bis 3, **dadurch gekennzeichnet, dass** der Flächeninhalt (A) des kreissektorförmigen Elements (3) zwischen 20 cm² und 200 cm² beträgt.

5. Vorlage (1) nach einem der Ansprüche 1 bis 4, **dadurch gekennzeichnet, dass** im Bereich des Mittelpunkts (M) des kreissektorförmigen Elements (3) eine Aussparung (9) vorgesehen ist.

6. Vorlage (1) nach Anspruch 5, **dadurch gekennzeichnet, dass** die Aussparung (9) kreisförmig ausgebildet ist mit einem Radius (r₂) von vorzugsweise 1 mm bis 11 mm.

7. Vorlage (1) nach einem der Ansprüche 1 bis 4, **dadurch gekennzeichnet, dass** im Bereich des Mittelpunkts (M) des kreissektorförmigen Elements (3) zumindest ein Einschnitt (10) vorgesehen ist.

8. Vorlage (1) nach Anspruch 7, **dadurch gekennzeichnet, dass** der zumindest eine Einschnitt (10) eine Tiefe (t) von 1 mm bis 11 mm aufweist.

9. Vorlage (1) nach einem der Ansprüche 1 bis 8, **dadurch gekennzeichnet, dass** das kreissektorförmige Element (3) aus hochabsorbierenden Kunststofffasern, insbesondere Acryl- oder Polyesterfasern, gebildet ist.

10. Vorlage (1) nach einem der Ansprüche 1 bis 9, **dadurch gekennzeichnet, dass** im kreissektorförmigen Element (3) eine Gelbildenden Substanz (11) angeordnet ist.

11. Vorlage (1) nach einem der Ansprüche 1 bis 10, **dadurch gekennzeichnet, dass** das kreissektorförmige Element (3) an der der flüssigkeitsdichten Schicht (5) gegenüberliegenden Seite ein Kontaktnetz (12) aufweist.

12. Vorlage (1) nach einem der Ansprüche 1 bis 11, **dadurch gekennzeichnet, dass** das Klebeelement (6) zum Befestigen des kreissektorförmigen Elements (3) in seiner Gebrauchslage durch eine Rundung gebildet ist.

13. Vorlage (1) nach einem der Ansprüche 1 bis 12, **dadurch gekennzeichnet, dass** die Klebeelemente (6, 7) einstückig ausgebildet sind.

14. Vorlage (1) nach einem der Ansprüche 1 bis 13, **dadurch gekennzeichnet, dass** die Dicke (d) des kreissektorförmigen Elements (3) 1 mm bis 9 mm, vorzugsweise 3 mm bis 3,5 mm beträgt.
